(19) Europäisches Patentamt
European Patent Office
Office européen des brevets

(11) **EP 2 527 819 A1**

(12) **EUROPEAN PATENT APPLICATION**
published in accordance with Art. 153(4) EPC

(43) Date of publication:
28.11.2012 Bulletin 2012/48

(51) Int Cl.:
**G01N 21/64** $^{(2006.01)}$ **G01N 15/14** $^{(2006.01)}$
**G01N 33/483** $^{(2006.01)}$

(21) Application number: 11734504.1

(22) Date of filing: 19.01.2011

(86) International application number:
**PCT/JP2011/000258**

(87) International publication number:
**WO 2011/089897 (28.07.2011 Gazette 2011/30)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: 21.01.2010 JP 2010010713

(71) Applicant: **Mitsui Engineering & Shipbuilding Co., Ltd.**
**Chuo-ku**
**Tokyo 104-8439 (JP)**

(72) Inventors:
• **HAYASHI, Hironori**
**Tamano-shi**
**Okayama 706-8651 (JP)**
• **NAKADA, Shigeyuki**
**Tamano-shi**
**Okayama 706-8651 (JP)**

(74) Representative: **Hards, Andrew**
**Global IP Europe**
**Patentanwaltskanzlei**
**Pfarrstrasse 14**
**80538 München (DE)**

(54) **METHOD FOR DETECTION OF FLUORESCENCE, PROCESS FOR PRODUCTION OF FLUORESCENT BEADS, AND FLUORESCENT BEADS**

(57) Two or more kinds of fluorescent beads containing at least two kinds of basic fluorochromes different in fluorescence intensity, fluorescence wavelength, and fluorescence relaxation time from each other, wherein a content ratio between the at least two kinds of basic fluorochromes and absolute amounts of contents of the basic fluorochromes are set so as to be different between different kinds of fluorescent beads. The fluorescent beads are used in a flow cytometer for fluorescence detection. This makes it possible to identify a greater variety of beads than before with high accuracy in a single measurement.

FIG.1

**Description**

Technical Field

The present invention relates to a fluorescence detecting method for identifying the kind of fluorescent bead by using fluorescence emitted from a fluorochrome-labeled fluorescent bead, a method for producing fluorochrome-labeled fluorescent beads, and fluorochrome-labeled fluorescent beads.

Background Art

In a flow cytometer used in medical and biological fields, fluorescence emitted from a fluorescent bead to which a biological substance binds by irradiation with laser light is detected and used to identify the kind of probe of the fluorescent bead to which the biological substance binds. In this case, the fluorescent bead has a probe (i.e., a substance having selective binding function such as a gene fragment having a complementary sequence or an antibody that binds to a specific protein) attached to the surface thereof.

Specifically, fluorescent beads stained and labeled with identifiable fluorochromes are mixed as fluorescent reagents into a suspension containing biological substances (e.g., cells, DNA, RNA, enzymes, proteins) labeled with a predetermined fluorochrome, and then the fluorescent beads are allowed to flow in a sheath fluid flowing through a tube of a flow cytometer under pressure at a speed of about 10 m/s to make a flow cell. Each of the fluorescent beads in the flow cell is irradiated with laser light to receive fluorescence emitted from the fluorescent bead and fluorescence emitted from the biological substance binding to the fluorescent bead. The kind of probe to which the biological substance binds can be identified by identifying these fluorescences and using the fluorescence emitted from the fluorescent bead as a label for identifying the kind of probe.

Particularly, it is possible to examine what kind of probe each of biological substances binds to by using fluorescent beads having different probes and stained with different identifiable fluorochromes depending on the kinds of probes. In this case, it is desired that a mixture of many kinds of fluorescent beads having different probes and a suspension containing biological substances is analyzed by a flow cytometer in a short period of time at one time.

As a conventional fluorescence detecting method, a method for identifying the kind of bead by using fluorescence emitted from a bead stained with a fluorochrome and having a probe attached to the surface thereof is known (Patent Literature 1). When fluorescence detection is performed by this method, beads previously stained with a mixture of two kinds of fluorochromes different in fluorescence relaxation time are used, and the kind of bead is identified by detecting fluorescence emitted from each of the beads and measuring the fluorescence relaxation time of the fluorescence.

Citation List

Patent Literature

Patent Literature 1: Japanese Patent No. 4300366

Summary of Invention

Technical Problem

It is considered that the above method makes it possible to identify several hundred kinds of beads in a single sequence of measurement. However, several hundred kinds of beads cannot be always identified with high accuracy in a single sequence of measurement of their fluorescence relaxation time.

In order to solve the above problem, it is an object of the present invention to provide a fluorescence detecting method capable of identifying a greater variety of beads than before with high accuracy in a single measurement, a method for producing fluorescent beads, and fluorescent beads.

Solution to Problem

An aspect of the present invention is provided with a fluorescence detecting method for identifying a kind of fluorescent bead by using fluorescence emitted from a fluorochrome-labeled fluorescent bead. The method includes the steps of:

measuring a fluorescence intensity and a fluorescence relaxation time per wavelength band by using two or more kinds of fluorescent beads labeled with two or more kinds of composite fluorochromes,

wherein the composite fluorochromes are different from each other, in a content ratio between two or more kinds of basic fluorochromes and in absolute amounts of contents of the basic fluorochromes, and

wherein the basic fluorochromes are different from each other, in fluorescence intensity, fluorescence wavelength and fluorescence relaxation time; and

identifying a kind of measured fluorescent bead by using information about the measured fluorescence intensity, the measured fluorescence relaxation time, and the wavelength band.

Specifically, the two kinds of basic fluorochromes are used, the fluorescence intensities of the basic fluorochromes are represented by $F_1$ and $F_2$, the fluorescence relaxation times of the basic fluorochromes are represented by $\tau_1$ and $\tau_2$, the content ratio between the basic fluorochromes is represented by x :(1-x) (x is a positive value less than 1), and constants determined by the absolute amounts of contents are represented by $\alpha_1$ and $\alpha_2$. Then, the content ratio and the absolute amounts of contents of each of the two or more kinds of composite fluorochromes are preferably set so that the two or more kinds of composite fluorochromes are different in composite fluorescence intensity F determined by the following formula (1) and composite fluorescence relaxation time $\tau$ determined by the following formula (2) from each other:

$$F = \alpha_1 \times \tau_1 \times x + \alpha_2 \times \tau_2 \times (1\text{-}x) \qquad (1)$$

$$\tau = \{\alpha_1 \times \tau_1^2 \times x + \alpha_2 \times \tau_2^2 \times (1\text{-}x)\}/F \qquad (2)$$

Another aspect of the present invention is provided with a method for producing two or more kinds of fluorochrome-labeled fluorescent beads. The method includes the steps of:

setting a content ratio between at least two kinds of basic fluorochromes and absolute amounts of contents of the basic fluorochromes so that different kinds of fluorescent beads are different in the content ratio and the absolute amounts of contents from each other,

wherein the basic fluorochromes are different in fluorescence intensity, fluorescence wavelength, and fluorescence relaxation time from each other; and labeling fluorescent beads with the composite fluorochromes obtained based on the set content ratio and the set absolute amounts of contents, respectively.

Specifically, the two kinds of basic fluorochromes are used, the fluorescence intensities of the basic fluorochromes are represented by $F_1$ and $F_2$, the fluorescence relaxation times of the basic fluorochromes are represented by $\tau_1$ and $\tau_2$, the content ratio between the basic fluorochromes is represented by x :(1-x) (x is a positive value less than 1), and constants determined by the absolute amounts of contents are represented by $\alpha_1$ and $\alpha_2$. Then, a fluorescence intensity F of each of the composite fluorochromes is preferably determined by the following formula (3) and a fluorescence relaxation time $\tau$ of each of the composite fluorochromes is preferably determined by the following formula (4):

$$F = \alpha_1 \times \tau_1 \times x + \alpha_2 \times \tau_2 \times (1\text{-}x) \qquad (3)$$

$$\tau = \{\alpha_1 \times \tau_1^2 \times x + \alpha_2 \times \tau_2^2 \times (1\text{-}x)\}/F \qquad (4)$$

Another aspect of the present invention is provided with two or more kinds of fluorochrome-labeled fluorescent beads including:

at least two kinds of basic fluorochromes different in fluorescence intensity, fluorescence wavelength, and fluorescence relaxation time from each other, wherein a content ratio between the at least two kinds of basic fluorochromes and absolute amounts of contents of the basic fluorochromes are set so as to be different between different kinds of fluorescent beads.

Advantageous Effects of Invention

The use of the fluorescence detecting method, the method for producing fluorescent beads, and the fluorescent beads according to the above aspects of the present invention makes it possible to identify a greater variety of beads with high accuracy in a single measurement.

Brief Description of Drawings

Fig. 1 is a schematic diagram illustrating the configuration of a flow cytometer using a fluorescence detecting apparatus according to an embodiment of the present invention.
Fig. 2 is a schematic diagram illustrating the configuration of one example of a light source unit used in the flow cytometer illustrated in Fig. 1.
Fig. 3 is a schematic diagram illustrating the configuration of one example of a light-receiving unit used in the flow cytometer illustrated in Fig. 1.
Fig. 4 is a schematic diagram illustrating the configuration of one example of a control·processing unit used in the flow cytometer illustrated in Fig. 1.
Fig. 5 is a schematic diagram illustrating the configuration of one example of an analyzing device used in the flow cytometer illustrated in Fig. 1.
Figs 6A to 6E are graphs for explaining data measured by the flow cytometer illustrated in Fig. 1.

Description of Embodiment

Hereinbelow, a fluorescence detecting method, a method for producing fluorescent beads, and fluorescent beads according to the present invention will be described in detail.

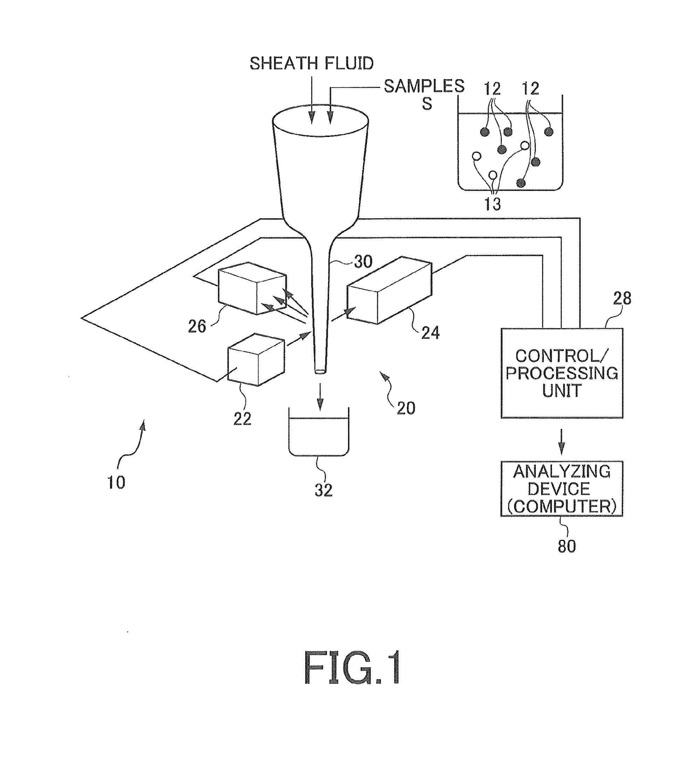

Fig. 1 is a schematic diagram illustrating the configuration of a flow cytometer 10 embodying the fluorescence detecting method according to the present invention.
In the flow cytometer 10, samples S each containing a fluorescent bead 12 and a biological substance 13 are allowed to flow together with a sheath fluid, and each of the samples S is irradiated with laser light whose intensity is modulated at a predetermined frequency when passing through a measurement point and fluorescence emitted from each sample S at this time is received and detected.
The fluorescent beads 12 are spherical, have a diameter of about several micrometers, and contain a composite fluorochrome. Each of the fluorescent beads 12 has a probe on its surface, and the kind of probe is different depending on the kind of composite fluorochrome. The different kinds of composite fluorochromes are different in fluorescence intensity and fluorescence relaxation time from each other. That is, the fluorescent beads 12 having different probes are labeled with different composite fluorochromes. The composite fluorochrome is produced using basic fluorochromes A to D different in fluorescence intensity, fluorescence wavelength, and fluorescence relaxation time from one another. The different kinds of composite fluorochromes are different in the content ratio among the basic fluorochromes A to D and the absolute amounts of contents of the basic fluorochromes A to D from each other.
On the other hand, the biological substances 13 contain a fluorochrome, such as a fluorescent protein, as a label.
The flow cytometer 10 includes a signal processing device (fluorescence detecting device) 20 and an analyzing device 80.
In the signal processing device 20, each of the samples S making a flow cell is irradiated with laser light, fluorescence emitted from the fluorochromes of the fluorescent bead 12 in the sample S and fluorescence emitted from the fluorochrome of the biological substance 13 binding to the probe of the fluorescent bead 12 are received by photoelectric converters, and fluorescence signals outputted from the photoelectric converters are processed.
The analyzing device 80 analyzes the biological substance binding to the probe of the fluorescent bead 12 from a processing result obtained by the signal processing device 20.
It is to be noted that the probe used in this embodiment is one having the property of (specifically) binding to a biological substance such as DNA or an antibody, but other examples of the probe used in the present invention include, but are not limited to, substances responsive to various measurement environment conditions such as PH and oxidation-reduction potential.
The signal processing device 20 has a laser light source unit 22, light-receiving units 24 and 26, a control·processing unit 28, and a tube 30. The control-processing unit 28 includes a control unit that modulates the intensity of laser light emitted from the laser light source unit 22 at a predetermined frequency and a signal processing unit that processes fluorescence signals from each of the samples S. The tube 30 allows the samples S to flow in a high-speed flow of sheath fluid to make a flow cell. A recovery container 32 is provided at the outlet of the tube 30. The flow cytometer 10 may include a cell sorter for separating the biological substances 13 binding to the fluorescent beads 12 in the samples S from the other biological substances 13 or the other fluorescent beads 12 by irradiation with laser light in a short period of time to sort the biological substances 13 into different recovery containers.

The laser light source unit 22 emits two laser beams different in wavelength. A lens system is provided to focus the laser beams on a predetermined position in the tube 30 so that a measurement point, at which each of the samples S is measured, is provided at this focus position.

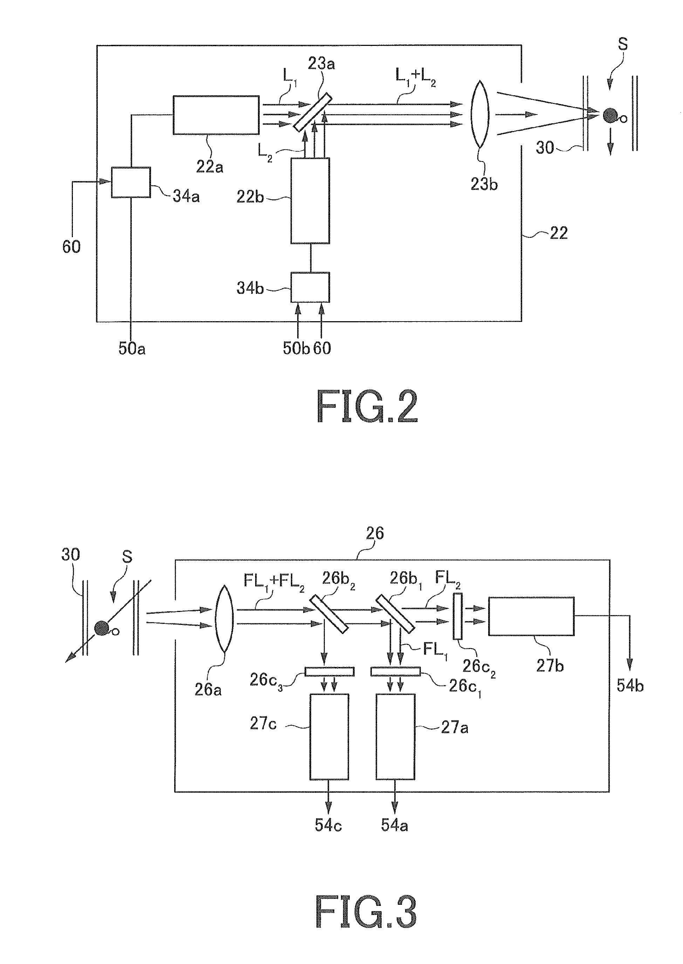

Fig. 2 is a diagram illustrating one example of the configuration of the laser light source unit 22. The laser light source unit 22 emits laser light which has a wavelength in the visible light range and whose intensity is modulated at a predetermined frequency.

The laser light source unit 22 has a light source 22a and a light source 22b. The light source 22a emits a laser beam whose wavelength is in the light absorption wavelength bands of the basic fluorochromes A and B and in the light absorption wavelength band of the fluorochrome of the biological substance 13. Therefore, the composite fluorochrome produced using the basic fluorochromes A and B and the fluorochrome attached to the biological substance 13 emit fluorescence having a predetermined fluorescence intensity and a predetermined fluorescence relaxation time at a predetermined wavelength band.

The light source 22b emits a laser beam whose wavelength is in the light absorption wavelength bands of the basic fluorochromes C and D. Therefore, the composite fluorochrome produced using the basic fluorochromes C and D emits fluorescence having a predetermined fluorescence intensity and a predetermined fluorescence relaxation time at a predetermined wavelength band.

The laser light source unit 22 has a dichroic mirror 23a, a lens system 23b, and laser drivers 34a and 34b. It is to be noted that a half mirror may be used instead of the dichroic mirror 23 a.

The dichroic mirror 23a transmits laser light of a specific wavelength band and reflects laser light of other wavelength bands. The lens system 23b focuses laser light $L_1 + L_2$ including a laser beam $L_1$ and a laser beam $L_2$ on the measurement point in the tube 30. The laser drivers 34a and 34b drive the light source 22a and the light source 22b, respectively.

As the light sources that emit laser light, for example, semiconductor lasers are employed. The output power of the laser light is, for example, about 5 to 100 mW.

On the other hand, the frequency (modulation frequency) used to modulate the intensity of each of the laser beams $L_1$ and $L_2$ has a period slightly longer than the fluorescence relaxation time(s), and is, for example, 10 to 50 MHz. The frequency used to modulate the intensity of the laser beam $L_1$ and the frequency used to modulate the intensity of the laser beam $L_2$ are different from each other. This allows fluorescences emitted from each of the samples S excited by the laser beams to have different frequencies, which makes it possible to know which of the laser beams has induced each of the fluorescences to separate information about the fluorescences.

The dichroic mirror 23a is a mirror that transmits the laser beam $L_1$ and reflects the laser beam $L_2$. The laser beam $L_1$ and the laser beam $L_2$ are mixed into one irradiation light by the dichroic mirror 23a, and each of the samples 12 is irradiated with the irradiation light at the measurement point.

The light source 22a oscillates at a predetermined wavelength band so that the fluorochromes are excited by the laser beam $L_1$ and emit fluorescence of specific wavelength bands. The light source 22b also oscillates at a predetermined wavelength band so that the fluorochromes are excited by the laser beam $L_2$ and emit fluorescence of specific wavelength bands. Each of the samples 12 to be excited by the laser beams $L_1$ and $L_2$ is irradiated with the laser beams $L_1$ and $L_2$ at the measurement point in the tube 30 when passing through the measurement point so that the fluorescent bead 12 emits fluorescence at a predetermined wavelength and/or the biological substance 13 emits fluorescence at a predetermined wavelength.

The light-receiving unit 24 and the laser light source unit 22 are arranged on opposite sides of the tube 30. The light-receiving unit 24 has a photoelectric converter that detects the laser light forward-scattered by the sample S passing through the measurement point and outputs a signal indicating that the sample S passes through the measurement point. The signal outputted from the light-receiving unit 24 is supplied to the control·processing unit 28 and the analyzing device 80 and used as a trigger signal that announces timing at which each sample S passes through the measurement point in the tube 30, an ON signal for starting processing, and an OFF signal.

On the other hand, the light-receiving unit 26 is arranged in a direction perpendicular to both a direction in which the laser light emitted from the laser light source unit 22 travels and a direction in which the samples S move in the tube 30. The light-receiving unit 26 has two or more photoelectric converters that receive fluorescence emitted from the sample S irradiated with the laser light at the measurement point. Fig. 3 is a schematic diagram illustrating the configuration of one example of the light-receiving unit 26.

The light-receiving unit 26 illustrated in Fig. 3 has a lens system 26a that focuses fluorescence signals from each of the samples S, dichroic mirrors $26b_1$ and $26b_2$, bandpass filters $26c_1$, $26c_2$, and $26c_3$, and photoelectric converters 27a, 27b, and 27c (light-receiving elements) such as photomultiplier tubes.

The lens system 26a is configured to focus fluorescence that has entered the light-receiving unit 26 onto light-receiving surfaces of the photoelectric converters 27a, 27b, and 27c.

Each of the dichroic mirrors $26b_1$ and $26b_2$ is a mirror that reflects fluorescence of a predetermined wavelength band and transmits fluorescence of other wavelength bands. The reflection wavelength band of each of the dichroic mirrors $26b_1$ and $26b_2$ and the transmission wavelength band of each of the bandpass filters $26c_1$, $26c_2$, and $26c_3$ are set so

that each of the photoelectric converters 27a, 27b, and 27c can receive fluorescence of a predetermined wavelength band after filtering by the bandpass filters $26c_1$, $26c_2$, and $26c_3$.

The bandpass filers $26c_1$, $26c_2$, and $26c_3$ are filters provided in front of the light-receiving surfaces of the photoelectric converters 27a, 27b, and 27c, respectively. Each of the bandpass filters $26c_1$, $26c_2$, and $26c_3$ transmits only fluorescence of a predetermined wavelength band. The wavelength bands of fluorescence that passes through the bandpass filters $26c_1$, $26c_2$, and $26c_3$ are set so as to correspond to the wavelength bands of fluorescence emitted from the basic fluorochromes A to D and the fluorochrome of the biological substance 13. Specifically, as the wavelength bands, a wavelength band $R_1$, a wavelength band $R_2$, and a wavelength band $R_3$ are set. The wavelength band $R_1$ is set to mainly receive fluorescence emitted from the fluorochromes of the fluorescent bead 12 by irradiation with the laser beam $L_1$ emitted from the light source 22a. The wavelength band $R_2$ is set to mainly receive fluorescence emitted from the fluorochromes of the fluorescent bead 12 by irradiation with the laser beam $L_2$ emitted from the light source 22b. The wavelength band $R_3$ is set to mainly receive fluorescence emitted from the fluorochrome of the biological substance 13 by irradiation with the laser beam $L_1$ emitted from the light source 22a. The wavelength band $R_1$ includes the wavelengths of fluorescence emitted from the composite fluorochrome, produced so as to contain the basic fluorochromes A and B, in the sample S irradiated with the laser beam $L_1$. The wavelength band $R_2$ includes the wavelengths of fluorescence emitted from the composite fluorochrome, produced so as to contain the basic fluorochromes C and D, in the sample S irradiated with the laser beam $L_2$.

Each of the photoelectric converters 27a, 27b, and 27c is a light-receiving element that has a sensor such as a photomultiplier tube and converts light received by its photoelectric surface into an electrical signal. Fluorescence received by each of the photoelectric converters is one excited by the laser beam whose intensity is modulated at a predetermined frequency, and therefore the intensity of a fluorescence signal outputted from each of the photoelectric converters also varies at the predetermined frequency. The fluorescence signal is supplied to the control·processing unit 28.

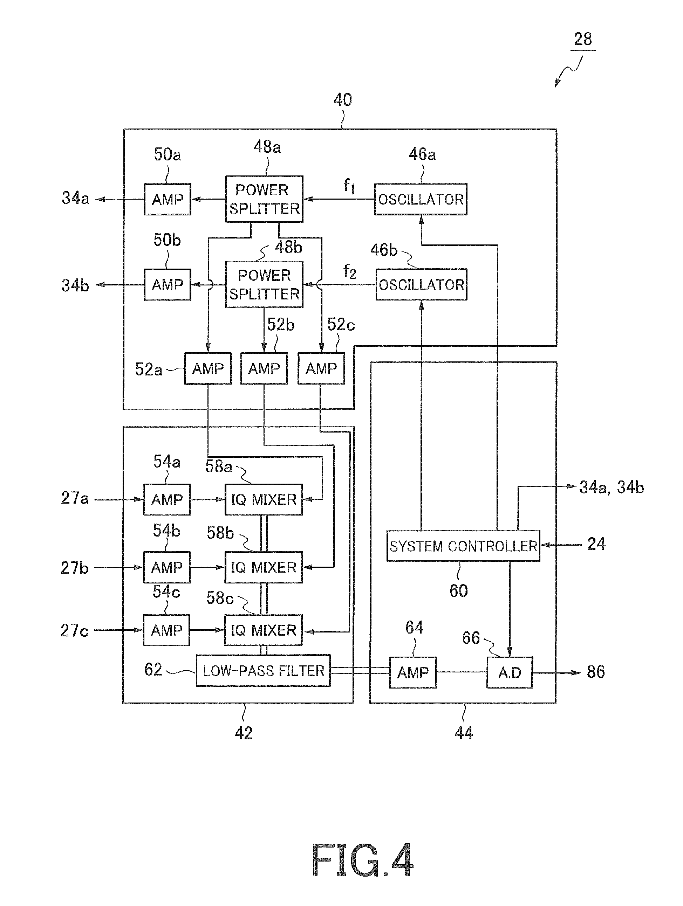

As illustrated in Fig. 4, the control·processing unit 28 has a signal generating section 40, a signal processing section 42, and a signal control section 44.

The signal generating section 40 generates a modulation signal for modulating the intensity of the laser beam $L_1$ at a predetermined frequency and a modulation signal for modulating the intensity of the laser beam $L_2$ at a predetermined frequency.

Specifically, the signal generating section 40 has oscillators 46a and 46b, power splitters 48a and 48b, and amplifiers 50a, 50b, 52a, 52b, and 52c. The signal generating section 40 supplies the generated modulation signals to the laser drivers 34a and 34b of the laser light source unit 22 and to the signal processing unit 42. As will be described later, the reason why the modulation signals are supplied to the signal processing section 42 is that they are used as reference signals for detecting the fluorescence signals outputted from the photoelectric converters 27a, 27b, and 27c. It is to be noted that each of the modulation signals is a signal obtained by adding, to a DC component, a sinusoidal signal with a predetermined frequency, and the frequency of each of the modulation signals is set to a value in the range of 10 to 50 MHz. The oscillator 46a oscillates to generate a signal with a frequency $f_1$ as a modulation signal and the oscillator 46b oscillates to generate a signal with a frequency $f_2$ as a modulation signal, and the frequency $f_1$ and the frequency $f_2$ are different from each other. It is to be noted that in this embodiment, a signal with a frequency $f_1$ and a signal with a frequency $f_2$ different from the frequency $f_{i1}$ are used as modulation signals, but when fluorescence to be measured can be sufficiently separated using the bandpass filters $26c_1$, $26c_2$, and $26c_3$, the frequency $f_1$ and the frequency $f_2$ do not need to be different from each other and may be the same.

Further, the oscillators 46a and 46b may be configured so that the frequency $f_1$ and the frequency $f_2$ can be freely changed. Further, the bandpass filters $26c_1$, $26c_2$, and $26c_3$ may be variable filters whose transmission wavelength bands can be changed depending on the kinds of fluorescent beads 12 used.

The signal processing section 42 is a section that extracts information about the fluorescence intensity and phase delay of fluorescence emitted by irradiation with the laser light by using the fluorescence signals outputted from the photoelectric converters 27a, 27b, and 27c. The signal processing section 42 has amplifiers 54a, 54b, and 54c that amplify the fluorescence signals outputted from the photoelectric converters 27a, 27b, and 27c, respectively, IQ mixers 58a, 58b, and 58c that combine each of the amplified fluorescence signals and the modulation signal that is a sinusoidal signal supplied from the signal generating section 40 as a reference signal, and a low-pass filter 62.

Each of the IQ mixers 58a, 58b, and 58c is a device that mixes the fluorescence signal supplied from one of the photoelectric converter 27a, 27b, or 27c and the modulation signal supplied from the signal generating section 40 as a reference signal. Specifically, each of the IQ mixers 58a, 58b, and 58c multiplies the reference signal and the fluorescence signal (RF signal) to generate a signal containing a component of the fluorescence signal which is in phase with the modulation signal, and a signal containing a component of the fluorescence signal which is phase-shifted by 90 degrees with respect to the modulation signal. The signal containing a component in phase with the modulation signal is generated by mixing the modulation signal and the fluorescence signal. The signal containing a component phase-shifted by 90 degrees with respect to the modulation signal is generated by mixing the fluorescence signal and a signal whose phase is shifted by 90 degrees with respect to the modulation signal.

The low-pass filter 62 filters a low-frequency signal of each of the signals generated by the IQ mixers 58a, 58b, and 58c. By performing such filtering, the component (Re component) of the fluorescence signal, which is in phase with the modulation signal, and the component (Im component) of the fluorescence signal, which is phase-shifted by 90 degrees with respect to the modulation signal, are extracted as fluorescence data. The extracted components are sent to the signal control section 44. The Re component and the Im component are obtained from each of the wavelength band $R_1$ set to correspond to the photoelectric converter 27a, the wavelength band $R_2$ set to correspond to the photoelectric converter 27b, and the wavelength band $R_3$ set to correspond to the photoelectric converter 27c. Therefore, a pair of the Re component and the Im component obtained from the wavelength band $R_1$, a pair of the Re component and the Im component obtained from the wavelength band $R_2$, and a pair of the Re component and the Im component obtained from the wavelength band $R_3$ are sent to the signal control section 44. Hereinafter, a process including mixing by the IQ mixers 58a, 58b, and 58c and filtering by the low-pass filter 62 is referred to as a frequency down conversion process, and data obtained by this process is referred to as fluorescence data.

The signal control section 44 amplifies the Re component and the Im component of each of the fluorescence signals sent from the signal processing section 42 and performs AD conversion.
Specifically, the signal control section 44 has a system controller 60 that provides directions for controlling the operation of each of the units and manages all the operations of the flow cytometer 10, an amplifier 64 that amplifies the Re component and the Im component generated by the signal processing section 42, and an A/D converter 66 that samples the amplified Re component and Im component.

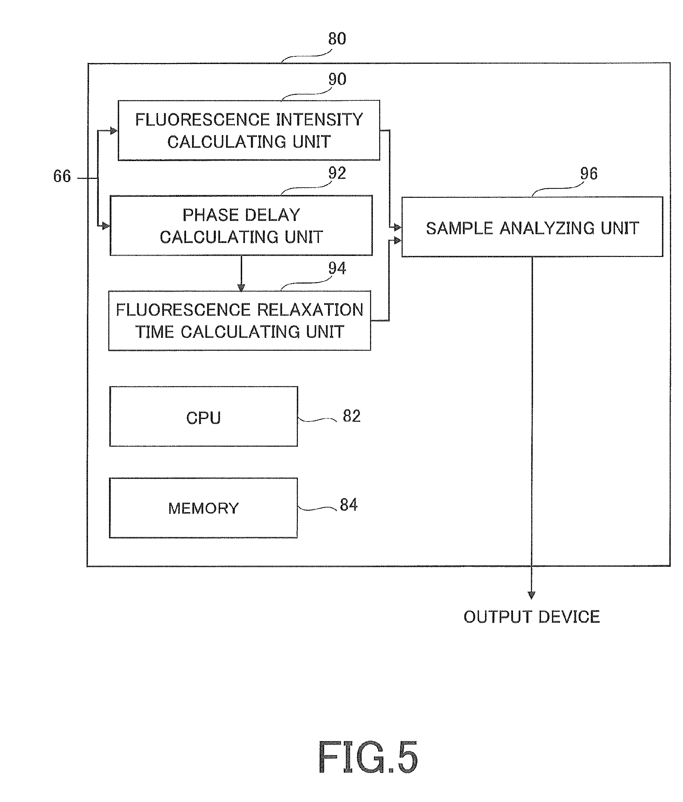

The analyzing device 80 determines the fluorescence intensity of the emitted fluorescence from the Re component and the Im component AD converted by the signal control section 44. Further, the analyzing device 80 determines the phase delay angle of the fluorescence with respect to the intensity modulation of the laser beam, and further determines a fluorescence relaxation time constant (fluorescence relaxation time) from the phase delay angle and a fluorescence intensity. Fig. 5 is a schematic diagram illustrating the configuration of the analyzing device 80.
The analyzing device 80 is constituted from a computer including a CPU 82 and a memory 84. The analyzing device 80 further includes a fluorescence intensity calculating unit 90, a phase delay calculating unit 92, a fluorescence relaxation time calculating unit 94, and a sample analyzing unit 96. Each of the units is a software module that performs its function by executing a program on the computer. These units may be, of course, implemented by dedicated circuits.

The fluorescence intensity calculating unit 90 determines the absolute value of a complex number from the fluorescence data supplied from the A/D converter 66 to calculate a fluorescence intensity.
The phase delay calculating unit 92 calculates, from the fluorescence data supplied from the A/D converter 66, the argument of a complex number whose real part is the Re component and imaginary part is the Im component ($\tan^{-1}$ (Im component of fluorescence data/Re component of fluorescence data)) as a phase delay θ.
The fluorescence relaxation time calculating unit 94 calculates a fluorescence relaxation time τ according to the formula: τ = 1/(2πf)·tan (θ) using the phase delay θ calculated by the phase delay calculating unit 92. Here, f is the frequency used to modulate the intensity of the laser beam $L_1$ or $L_2$. The reason why the fluorescence relaxation time τ can be calculated according to the formula: τ = 1/(2πf}·tan (θ) is that a fluorescence phenomenon shows a change in accordance with a primary relaxation process.
The sample analyzing unit 96 analyzes which kind of fluorescent bead 12 the biological substance 13 binds to by using the calculated fluorescence intensity and fluorescence relaxation time τ. The sample analyzing unit 96 outputs the result of analysis and data and graphs generated for analysis to an output device such as a printer or a display device.

Specifically, the sample analyzing unit 96 identifies fluorescence emitted from the fluorescent bead 12 based on the fluorescence intensity, the fluorescence relaxation time, and the fluorescence wavelength band and further identifies fluorescence emitted from the biological substance 13. At this time, the sample analyzing unit 96 determines whether or not the fluorescence emitted from the biological substance 13 has been received at the same time as the fluorescence emitted from the fluorescent bead 12 when the kind of fluorescent bead 12 is identified from the identified fluorescence. Further, the sample analyzing unit 96 determines which kind of fluorescent bead 12 has emitted fluorescence received at the same time as the fluorescence emitted from the biological substance 13.
This makes it possible to know which kind of fluorescent bead 12 the biological substance 13 has bound to and therefore to analyze the characteristics of the biological substance 13.

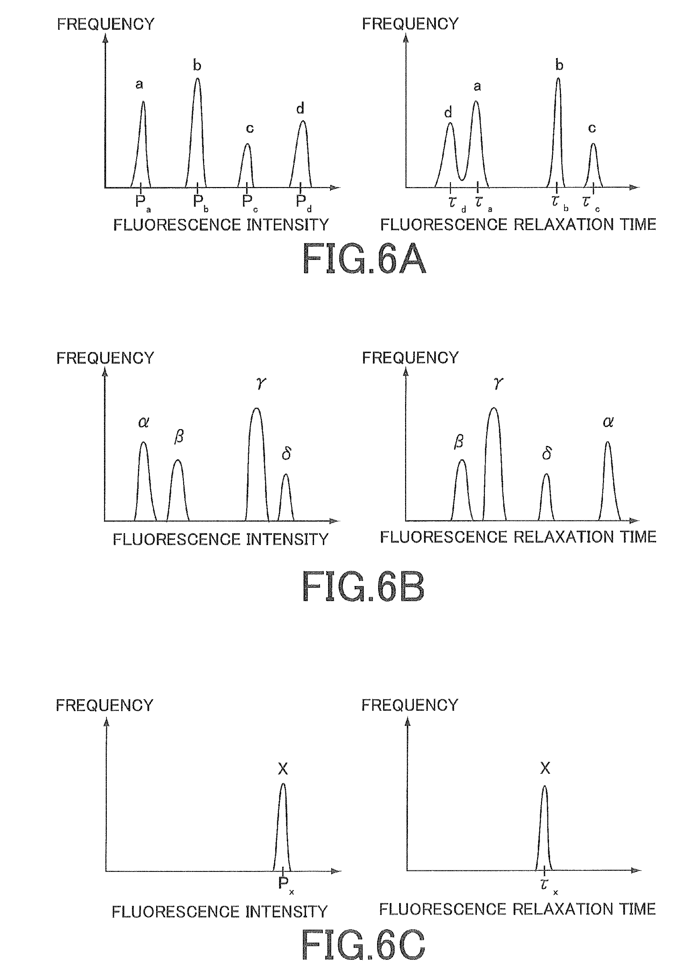

Fig. 6A illustrates one example of a histogram of the fluorescence intensity of fluorescence measured in the wavelength band $R_1$ and one example of a histogram of the fluorescence relaxation time of the fluorescence. Fig. 6B illustrates one example of a histogram of the fluorescence intensity of fluorescence measured in the wavelength band $R_2$ and one example of a histogram of the fluorescence relaxation time of the fluorescence.
In the wavelength band $R_1$, four kinds of fluorescence a to d emitted from the fluorescent beads 12 are measured. The fluorescent beads 12 include two or more kinds of beads different in the content ratio between the basic fluorochromes A and D and the absolute amounts of contents of the basic fluorochromes A and D, and the different kinds of fluorescent beads 12 emit different fluorescence. The fluorescence a to d measured in the wavelength band $R_1$ are different in

fluorescence intensity and fluorescence relaxation time, and therefore the four kinds of fluorescence can be identified by the histograms illustrated in Fig. 6A. Even when the fluorescence relaxation time of the fluorescence a and the fluorescence relaxation time of the fluorescence d are close to each other in Fig. 6A, the fluorescence a and the fluorescence d can be separated or distinguished from each other because there is a sufficient distance between the distribution of intensity of the fluorescence a and the distribution of intensity of the fluorescence d in the histogram of fluorescence intensity.

Also in the wavelength band $R_2$, four kinds of fluorescence $\alpha$ to $\delta$ emitted from the fluorescent beads 12 are measured. The fluorescences $\alpha$ to $\delta$ measured in the wavelength band $R_2$ are different in fluorescence intensity and fluorescence relaxation time. Therefore, the four kinds of fluorescence can be identified by the histograms illustrated in Fig. 6B. Even when the fluorescence relaxation time of the fluorescence $\beta$ and the fluorescence relaxation time of the fluorescence $\gamma$ are close to each other in Fig. 6B, the fluorescence $\beta$ and the fluorescence $\gamma$ can be separated or distinguished from each other because there is a sufficient distance between the distribution of intensity of the fluorescence $\beta$ and the distribution of intensity of the fluorescence $\gamma$ in the histogram of fluorescence intensity.

In the wavelength band $R_3$, fluorescence emitted from the biological substances 13 is measured. The fluorescence can be identified by the histograms illustrated in Fig. 6C.

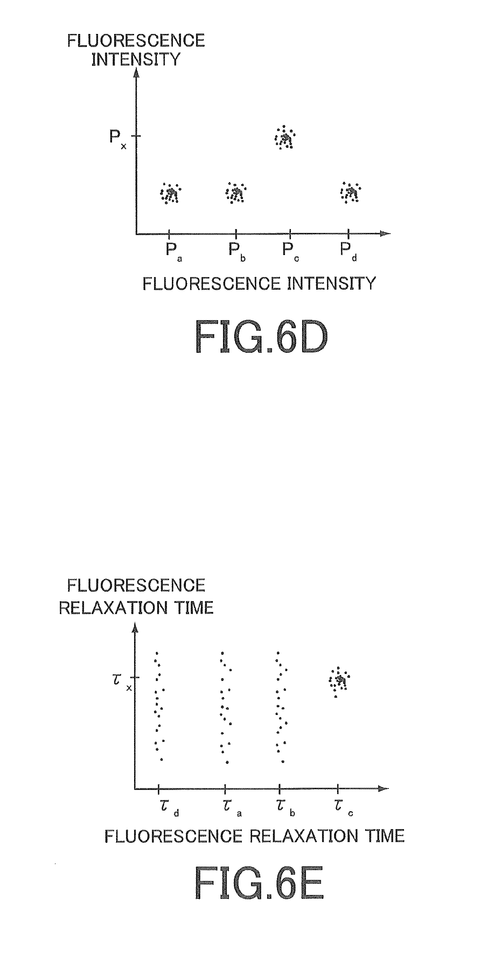

Fig. 6D is a scatter diagram obtained by plotting data about fluorescence intensity P to investigate the correspondence between the fluorescence intensity P of fluorescence measured in the wavelength band $R_1$ and the fluorescence intensity P of fluorescence measured in the wavelength band $R_3$. Fig. 6E is a scatter diagram obtained by plotting data about fluorescence relaxation time $\tau$ to investigate the correspondence between the fluorescence relaxation time $\tau$ of fluorescence measured in the wavelength band $R_1$ and the fluorescence relaxation time $\tau$ of fluorescence measured in the wavelength band $R_3$.

Fig. 6D indicates that when fluorescence data about the fluorescence intensity $P_c$ of fluorescence measured in the wavelength band $R_1$ is obtained, fluorescence data about the fluorescence intensity $P_x$ of fluorescence measured in the wavelength band $R_3$ is obtained at the same time. Fig. 6E indicates that when fluorescence data about the fluorescence relaxation time $\tau_c$ of fluorescence measured in the wavelength band $R_1$ is obtained, fluorescence data about the fluorescence relaxation time $\tau_x$ of fluorescence measured in the wavelength band $R_3$ is obtained at the same time.

Therefore, in this embodiment, it is possible to identify which kind of fluorescent bead 12 the biological substance 13 is likely to bind to by determining whether or not fluorescence data including the fluorescence relaxation time $\tau_x$ of fluorescence measured in the wavelength band $R_3$ is obtained at the same time as fluorescence emitted from the fluorescent bead 12 is measured.

In this embodiment described above, as illustrated in Fig. 6A, the fluorescent beads 12 that emit different kinds of fluorescence can be achieved by using three identifying factors, wavelength bands $R_1$ and $R_2$, fluorescence intensity, and fluorescence relaxation time. Such two or more kinds of fluorescent beads 12 can be achieved by, for example, labeling with three or more kinds of composite fluorochromes different in the content ratio between two kinds of basic fluorochromes A and B, which are different in fluorescence intensity, fluorescence wavelength, and fluorescence relaxation time from each other, and the absolute amounts of contents of the basic fluorochromes A and B from one another. In this embodiment, two kinds of basic fluorochromes A and B are used, but three of more kinds of basic fluorochromes may be used.

For example, two kinds of wavelength bands (wavelength band 1 and wavelength band 2) are assumed to be set so that many kinds of fluorescence emitted from the fluorescent beads 12 are received in the wavelength band 1 and the wavelength band 2 to determine the fluorescence intensity and fluorescence relaxation time of each fluorescence. Then, 625 (= 5 x 5 x 5 x 5) kinds of fluorescent beads 12 identifiable by the flow cytometer 10 can be obtained in total by setting 5 kinds of fluorescence relaxation times for the wavelength band 1, setting 5 kinds of fluorescence relaxation times for the wavelength band 2, setting 5 kinds of fluorescence intensities for the wavelength band 1, and setting 5 kinds of fluorescence intensities for the wavelength band 2. That is, when containing a basic fluorochrome that emits fluorescence received in the wavelength band 1 and a basic fluorochrome that emits fluorescence received in the wavelength band 2, the fluorescent beads 12 can be identified using both the measurement result obtained from the wavelength band 1 and the measurement result obtained from the wavelength band 2. In this case, the wavelength band 1 and the wavelength band 2 are preferably set to, for example, a short-wavelength band (450 to 500 nm) and a long-wavelength band (600 to 650 mm) separated from each other.

Specifically, two kinds of basic fluorochromes are assumed to be used. Then, the fluorescence intensities of the basic fluorochromes are represented by $F_1$ and $F_2$, the fluorescence relaxation times of the basic fluorochromes are represented by $\tau_1$ and $\tau_2$, the content ratio between the basic fluorochromes is represented by x: (1-x) (x is a positive value less than 1), and constants (pre-exponential factors) determined by the absolute amounts of contents of the basic fluorochromes are represented by $\alpha_1$ and $\alpha_2$. In this case, the content ratio and the absolute amounts of contents are preferably set so that three or more kinds of fluorescent beads produced are different in fluorescence intensity F determined by the following formula (1) and fluorescence relaxation time $\tau$ determined by the following formula (2) from one another.

$$F = \alpha_1 \times \tau_1 \times x + \alpha_2 \times \tau_2 \times (1\text{-}x) \qquad (1)$$

$$\tau = \{\alpha_1 \times \tau_1^2 \times x + \alpha_2 \times \tau_2^2 \times (1\text{-}x)\}/F \qquad (2)$$

The constants $\alpha_1$ and $\alpha_2$ are values that are determined by the absolute amounts of contents and increase as the absolute amounts of contents increase. Therefore, the fluorescence intensity of fluorescence emitted from a composite fluorochrome can be increased by increasing the absolute amounts of contents. On the other hand, the fluorescence relaxation time varies depending on the content ratio of x:(1-x) even when the constants $\alpha_1$ and $\alpha_2$ are increased. Therefore, the fluorescence intensity and the fluorescence relaxation time can be set independently of each other.

The fluorescent beads 12 are produced, for example, in the following manner. The content ratio between at least two kinds of basic fluorochromes different in fluorescence intensity, fluorescence wavelength, and fluorescence relaxation time from each other and the absolute amounts of contents of the basic fluorochromes are set so that the fluorescence intensity and fluorescence relaxation time of a composite fluorochrome obtained based on the content ratio and the absolute amounts of contents are different from those of the basic fluorochromes and from those of other composite fluorochromes. Then, the fluorescent beads 12 are labeled with the composite fluorochrome obtained based on the set content ratio and the set absolute amounts of contents. For example, the fluorescent beads 12 may be labeled by coating the surface of the fluorescent beads 12 with the composite fluorochrome. Alternatively, the fluorescent beads 12 may be labeled by directly adding the composite fluorochrome to the material of the fluorescent beads 12 and then forming the material into spheres.

When two kinds of basic fluorochromes are used, the fluorescence intensities of the basic fluorochromes are represented by $F_1$ and $F_2$, the fluorescence relaxation times of the basic fluorochromes are represented by $\tau_1$ and $\tau_2$, the content ratio between the basic fluorochromes is represented by x: (1-x) (x is a positive value less than 1), and constants determined by the absolute amounts of contents of the basic fluorochromes are represented by $\alpha_1$ and $\alpha_2$, the fluorescence intensity F and fluorescence relaxation time $\tau$ of a composite fluorochrome are determined by the above formulas (1) and (2), respectively.

The fluorescence detecting apparatus, the fluorescence detecting method, and the fluorescence signal processing method according to the present invention have been described above in detail, but the present invention is not limited to the above embodiment. It is apparent that various changes and modifications may be made without departing from the scope of the present invention.

Reference Signs List

| | |
|---|---|
| 10 | flow cytometer |
| 12 | fluorescent bead |
| 20 | signal processing device |
| 22 | laser light source unit |
| 22a, 22b | light source |
| 23a, 26b | dichroic mirror |
| 23b, 26a | lens system |
| 24, 26 | light-receiving unit |
| $26c_1$, $26c_2$, $26c_3$ | bandpass filter |
| 27a, 27b, 27c | photoelectric converter |
| 28 | control·processing unit |
| 30 | tube |
| 32 | recovery container |
| 34a, 34b | laser driver |
| 48a, 48b | power splitter |
| 40 | signal generating section |
| 42 | signal processing section |
| 44 | signal control section |

| 46a, 46b | oscillator |
| 50a, 50b, 52a, 52b, 52c, 54a, 54b, 54c, 64 | amplifier |
| 58a, 58b, 58c | IQ mixer |
| 60 | system controller |
| 62 | low-pass filter |
| 66 | A/D converter |
| 80 | analyzing device |
| 82 | CPU |
| 84 | memory |
| 90 | fluorescence intensity calculating unit |
| 92 | phase delay calculating unit |
| 94 | fluorescence relaxation time calculating unit |
| 96 | sample analyzing unit |

**Claims**

1.  A fluorescence detecting method for identifying a kind of fluorescent bead by using fluorescence emitted from a fluorochrome-labeled fluorescent bead, the method comprising the steps of:

    measuring a fluorescence intensity and a fluorescence relaxation time per wavelength band by using two or more kinds of fluorescent beads labeled with two or more kinds of composite fluorochromes,
    wherein the composite fluorochromes are different from each other in a content ratio between two or more kinds of basic fluorochromes and in absolute amounts of contents of the basic fluorochromes, and
    wherein the basic fluorochromes are different from each other in fluorescence intensity, fluorescence wavelength and fluorescence relaxation time; and
    identifying a kind of measured fluorescent bead by using information about the measured fluorescence intensity, the measured fluorescence relaxation time and the wavelength band.

2.  The fluorescence detecting method according to claim 1, wherein, when the two kinds of basic fluorochromes are used, the fluorescence intensities of the basic fluorochromes are represented by $F_1$ and $F_2$, the fluorescence relaxation times of the basic fluorochromes are represented by $\tau_1$ and $\tau_2$, the content ratio between the basic fluorochromes is represented by x :(1-x) (x is a positive value less than 1), and constants determined by the absolute amounts of contents are represented by $\alpha_1$ and $\alpha_2$, the content ratio and the absolute amounts of contents of each of the two or more kinds of composite fluorochromes are set so that the two or more kinds of composite fluorochromes are different in composite fluorescence intensity F determined by the following formula (1) and composite fluorescence relaxation time $\tau$ determined by the following formula (2) from each other:

$$F = \alpha_1 \times \tau_1 \times x + \alpha_2 \times \tau_2 \times (1\text{-}x) \quad (1)$$

$$\tau = \{\alpha_1 \times \tau_1^2 \times x + \alpha_2 \times \tau_2^2 \times (1\text{-}x)\}/F \quad (2)$$

3.  The fluorescence detecting method according to claim 1 or 2, wherein the fluorescence relaxation time is measured by receiving fluorescence emitted from each of the fluorescent beads when each of the fluorescent beads is irradiated with laser light whose intensity is modulated at a predetermined frequency and determining a phase delay angle of the received fluorescence with respect to intensity modulation of the laser light.

4.  The fluorescence detecting method according to claim 3,
    wherein the laser light that irradiates each of the fluorescent beads is mixed light of two or more laser beams different in wavelength, and
    wherein each of the two or more kinds of basic fluorochromes has a light absorption wavelength band including any one of the wavelengths of the laser beams.

5.  The fluorescence detecting method according to claim 3 or 4,

wherein each of the fluorescent beads has a probe operable to bind to one of specific substances,

wherein each of the specific substances contains a fluorochrome that emits fluorescence at a specific wavelength by irradiation with the laser light, and

receiving, when the fluorescence intensity and the fluorescence relaxation time are measured, the fluorescence emitted from the one of the specific substances as light whose wavelength band is different from fluorescence emitted from the fluorescent beads, and

identifying the one of the specific substances by determining the fluorescence intensity and fluorescence relaxation time thereof.

6. The fluorescence detecting method according to claim 5, wherein

when a kind of measured fluorescent beads is identified,

obtaining per wavelength a kind of a scatter diagram of fluorescence intensity, the scatter diagram representing a correspondence between the fluorescence intensities of fluorescence emitted from the specific substances and the fluorescence intensities of fluorescence emitted from the fluorescent beads and

obtaining per wavelength a scatter diagram of fluorescence relaxation time, the scatter diagram representing a correspondence between the fluorescence relaxation times of fluorescence emitted from the specific substances and the fluorescence relaxation times of fluorescence emitted from the fluorescent beads, and

identifying by using the obtained scatter diagrams a kind of fluorescent bead to which each of the specific substances binds.

7. The fluorescence detecting method according to any one of claims 1 to 6,

wherein the fluorescent beads flow, one by one, along a flow path so as to pass through a measurement point on which the laser light is focused and

measuring, when each of the fluorescent beads passes through the measurement point, the fluorescence intensity and the fluorescence relaxation time.

8. A method for producing two or more kinds of fluorochrome-labeled fluorescent beads, the method comprising steps of:

setting a content ratio between at least two kinds of basic fluorochromes and absolute amounts of contents of the basic fluorochromes so that different kinds of fluorescent beads are different in the content ratio and the absolute amounts of contents from each other,

wherein the basic fluorochromes are different in fluorescence intensity, fluorescence wavelength and fluorescence relaxation time from each other; and

labeling fluorescent beads with the composite fluorochromes obtained based on the set content ratio and the set absolute amounts of contents, respectively.

9. The method for producing fluorescent beads according to claim 3, wherein, when the two kinds of basic fluorochromes are used, the fluorescence intensities of the basic fluorochromes are represented by $F_1$ and $F_2$, the fluorescence relaxation times of the basic fluorochromes are represented by $\tau_1$ and $\tau_2$, the content ratio between the basic fluorochromes is represented by x :(1-x) (x is a positive value less than 1), and constants determined by the absolute amounts of contents are represented by $\alpha_1$ and $\alpha_2$, a fluorescence intensity F of each of the composite fluorochromes is determined by the following formula (3) and a fluorescence relaxation time $\tau$ of each of the composite fluorochromes is determined by the following formula (4):

$$F = \alpha_1 \times \tau_1 \times x + \alpha_2 \times \tau_2 \times (1\text{-}x) \quad (3)$$

$$\tau = \{\alpha_1 \times \tau_1^2 \times x + \alpha_2 \times \tau_2^2 \times (1\text{-}x)\}/F \quad (4)$$

10. Two or more kinds of fluorochrome-labeled fluorescent beads comprising:

at least two kinds of basic fluorochromes different in fluorescence intensity, fluorescence wavelength and fluorescence relaxation time from each other,

wherein a content ratio between the at least two kinds of basic fluorochromes and absolute amounts of contents of the basic fluorochromes are set so as to be different between different kinds of fluorescent beads.

**11.** The fluorescent beads according to claim 10, wherein each of which has a probe operable to bind to a substance which varies according to a kind of fluorescent bead.

FIG.1

## FIG.2

## FIG.3

FIG.4

80

90

FLUORESCENCE INTENSITY
CALCULATING UNIT

66

92

PHASE DELAY
CALCULATING UNIT

96

SAMPLE ANALYZING UNIT

94

FLUORESCENCE RELAXATION
TIME CALCULATING UNIT

CPU

82

MEMORY

84

OUTPUT DEVICE

# FIG.5

FREQUENCY

a
b
c
d

P$_a$  P$_b$  P$_c$  P$_d$

FLUORESCENCE INTENSITY

FREQUENCY

d
a
b
c

$\tau_d$  $\tau_a$  $\tau_b$  $\tau_c$

FLUORESCENCE RELAXATION TIME

FIG.6A

FREQUENCY

$\alpha$
$\beta$
$\gamma$
$\delta$

FLUORESCENCE INTENSITY

FREQUENCY

$\beta$
$\gamma$
$\delta$
$\alpha$

FLUORESCENCE RELAXATION TIME

FIG.6B

FREQUENCY

X

P$_x$

FLUORESCENCE INTENSITY

FREQUENCY

X

$\tau_x$

FLUORESCENCE RELAXATION TIME

FIG.6C

FLUORESCENCE
INTENSITY

FLUORESCENCE INTENSITY

# FIG.6D

FLUORESCENCE
RELAXATION TIME

FLUORESCENCE RELAXATION TIME

# FIG.6E

| | INTERNATIONAL SEARCH REPORT | International application No. |
|---|---|---|
| | | PCT/JP2011/000258 |

A. CLASSIFICATION OF SUBJECT MATTER
*G01N21/64*(2006.01)i, *G01N15/14*(2006.01)i, *G01N33/483*(2006.01)i

According to International Patent Classification (IPC) or to both national classification and IPC

B. FIELDS SEARCHED

Minimum documentation searched (classification system followed by classification symbols)
G01N21/00-21/74, G01N15/14, G01N33/45-33/98

Documentation searched other than minimum documentation to the extent that such documents are included in the fields searched
| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)
JSTPlus/JMEDPlus/JST7580(JDreamII)

C. DOCUMENTS CONSIDERED TO BE RELEVANT

| Category* | Citation of document, with indication, where appropriate, of the relevant passages | Relevant to claim No. |
|---|---|---|
| Y | JP 2006-275858 A  (National Institute of Advanced Industrial Science and Technology), 12 October 2006 (12.10.2006), paragraphs [0009] to [0041]; fig. 1 to 8 (Family: none) | 1-11 |
| Y | JP 2007-127415 A  (Mitsui Engineering & Shipbuilding Co., Ltd.), 24 May 2007 (24.05.2007), paragraphs [0012] to [0014], [0024] to [0030] & US 2009/0012721 A1    & EP 1855102 A1 & WO 2006/088047 A1 | 1-11 |

☒  Further documents are listed in the continuation of Box C.          ☐  See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 28 March, 2011 (28.03.11) | 05 April, 2011 (05.04.11) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

| | International application No. |
|---|---|
| | PCT/JP2011/000258 |

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 2003-083894 A  (Sumitomo Electric Industries, Ltd.), 19 March 2003 (19.03.2003), paragraphs [0043], [0053] to [0071]; fig. 1 to 13 (Family: none) | 1-11 |
| Y | JP 2006-266905 A  (Mitsui Engineering & Shipbuilding Co., Ltd.), 05 October 2006 (05.10.2006), paragraphs [0081], [0096] to [0098] (Family: none) | 1-11 |
| A | JP 2009-210379 A  (Mitsui Engineering & Shipbuilding Co., Ltd.), 17 September 2009 (17.09.2009), entire text; all drawings & EP 2251672 A1        & WO 2009/110314 A1 | 1-11 |
| A | JP 2006-275905 A  (National Institute of Advanced Industrial Science and Technology), 12 October 2006 (12.10.2006), entire text; all drawings (Family: none) | 1-11 |
| A | JP 2006-284231 A  (National Institute of Advanced Industrial Science and Technology), 19 October 2006 (19.10.2006), entire text; all drawings (Family: none) | 1-11 |
| A | WO 2008/042565 A2  (GLAXO GLOUP LTD.), 10 April 2008 (10.04.2008), entire text; all drawings & JP 2010-505126 A      & US 2010/0148091 A & EP 2081830 A | 1-11 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4300366 B